(19) Europäisches Patentamt
European Patent Office
Office européen des brevets

(11) **EP 3 415 089 A1**

(12) **EUROPEAN PATENT APPLICATION**

(43) Date of publication:
**19.12.2018 Bulletin 2018/51**

(51) Int Cl.:
*A61B 5/0484* (2006.01)    *A61B 5/00* (2006.01)

(21) Application number: **17305705.0**

(22) Date of filing: **12.06.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **RYTHM**
**75009 Paris (FR)**

(72) Inventors:
• **MERCIER, Hugo**
**75010 PARIS (FR)**

• **SOULET DE BRUGIERE, Quentin**
**75001 PARIS (FR)**
• **KALIFA, Jérôme**
**92160 ANTONY (FR)**
• **PINAUD, Clémence**
**75014 PARIS (FR)**
• **DEHAENE, David**
**75002 PARIS (FR)**

(74) Representative: **Cabinet Plasseraud**
**66, rue de la Chaussée d'Antin**
**75440 Paris Cedex 09 (FR)**

(54) **METHOD AND SYSTEM FOR COMMANDING THE PRODUCTION OF AN ACOUSTIC WAVEFORM BASED ON A PHYSIOLOGICAL CONTROL SIGNAL, AND ASSOCIATED COMPUTER PROGRAM**

(57)     The method for commanding the production of an acoustic waveform based on a physiological control signal, comprises:
- one provides sampled sound data comprising S sound samples stored on a data carrier,
- repeatedly, for n successive respective time intervals $[t_n; t_{n+1}[$ between an initial time $t_a$ and a final time $t_b$:
. one provides a physiological control signal phi(t) as a function of time, during the current time interval $[t_n; t_{n+1}[$,
. a rate determination module (13) of a processor determines a rate $r_n$ of samples to be played during that time interval based on a value phi $(t_n)$ of the physiological control signal at time $t_n$,

. a command module (15) of the processor commands the play of a part of the acoustic waveform from the sampled sound data as a function of the determined rate $r_n$ of samples.

FIG. 1

**Description**

The invention relates to methods, systems and computer programs to command the production of acoustic waveforms.

More precisely, the invention relates to methods to command the production of acoustic waveforms. Acoustic waveforms are well known to be produced in a number of industries. The typical production of an acoustic waveform is performed by sending electric control signals to a transducer to be transformed into mechanical vibration, and hence sounds. These control signals are currently often stored in digital format as a sound file on a data carrier. These control signals are then sent electrically to a transducer which converts electrical signals into mechanical waves which creates sound.

In the sound industry, it is known to command the production of acoustic waveforms by handling the digital sound data. This is used for example to smoothly transition from one sound file to one another. This is used for example for the continuous production of music from a plurality of files.

Recently, it was proposed a wearable which generates acoustic sound waves to assist the user's sleep. An example of such a wearable can be found in WO 2016/083,598. To do so, the generated acoustic sound waves are user specific. There is a need for an improved wearable which would assist the user's sleep by generating user-specific acoustic waves.

According to a first object, the invention relates to a method for commanding the production of an acoustic waveform based on a physiological control signal, comprising:

-    providing sampled sound data comprising S sound samples stored on a data carrier,
-    repeatedly, for n successive respective time intervals $[t_n; t_{n+1}[$ between an initial time $t_a$ and a final time $t_b$:

   . providing a physiological control signal phi(t) as a function of time, during the current time interval $[t_n; t_{n+1}[$,
   . a rate determination module of a processor determines a rate $r_n$ of samples to be played during that time interval based on a value $phi(t_n)$ of the physiological control signal at time $t_n$,
   . a command module of the processor commands the play of a part of the acoustic waveform from the sampled sound data as a function of the determined rate $r_n$ of samples.

Thanks to these features, user-specific smooth generation of acoustic sound waves can be proposed. This would enable to assist the user's sleep.

According to some embodiments, one may use one or more of the following features:

-    the physiological control signal phi(t) is representative of the phase of a pseudo-periodic physiological signal;

-    the pseudo-periodic physiological signal is a respiratory signal of a user;

-    the pseudo-periodic physiological signal is an electro-encephalogram signal of a user;

-    the physiological control signal phi(t) is a strictly monotonous signal along time;

-    the rate $r_n$ of samples to be played is proportional to a speed (R) of number of played samples per time, between the initial time $t_a$ and the final time $t_b$;

-    the rate $r_n$ of samples to be played is proportional to an inverse of d.phi(t)/dt, where an operator (d.X/dt) designates a time derivative of signal X;

-    the command module of the processor re-samples N sound samples of the sound data into P sound samples, where P is lower than N and r=P/N;

-    at least one time interval, and notably all time intervals, have a time duration $\delta t = t_{n+1} - t_n$ at most equal to 5%, notably at most equal to 1%, preferably at most equal to 0.3%, notably at most equal to 0.1% of $t_b - t_a$;

-    the method for commanding the production of an acoustic waveform further comprises determining the physiological control signal phi(t) as a function of time based on a measurement by a sensor;

-    the physiological control signal phi(t) is determined from a pseudo-periodic physiological signal of period $t_b - t_a$;

- the method is repeated for further periods of time until the sample sound data is played.

According to another aspect, the invention relates to a method for producing an acoustic waveform comprising:

- applying the above method for commanding the production of an acoustic waveform,
- for each of said successive time intervals, playing a part of the acoustic waveform from the sampled sound data as a function of the determined rate $r_n$ of samples.

According to another aspect, the invention relates to a computer program comprising instructions for executing the steps of the above methods, when the computer program is run on a processor.

According to another aspect, the invention relates to a system for commanding the production of an acoustic waveform based on a physiological control signal, comprising:

- a data carrier storing sampled sound data comprising S sound samples,
- a processor configured to repeatedly, for n successive respective time intervals $[t_n; t_{n+1}[$ between the initial time $t_a$ and the final time $t_b$:

  a physiological control signal phi(t) being provided as a function of time, during the current time interval $[t_n; t_{n+1}[$,

  • determine a rate $r_n$ of samples to be played during that time interval based on a value phi $(t_n)$ of the physiological control signal at time $t_n$, using a rate determination module of the processor,
  • command the play of a part of the acoustic waveform from the sampled sound data as a function of the determined rate $r_n$ of samples, using a command module of the processor.

According to another aspect, the system for commanding the production of an acoustic waveform further comprises a wearable device adapted to be worn on the head of a user, housing the processor and data carrier, and comprising sensors to determine the physiological control signal phi(t) as a function of time.

The list of drawings hereby follows:

- Figure 1 is a schematic view of a self-contained device for stimulating brain waves that is worn on the head of a person, according to one embodiment of the invention,

- Figure 2 is a detail perspective view of a self-contained device for stimulating brain waves according to one embodiment of the invention, where the device comprises in particular first and second acoustic transducers respectively adapted to emit acoustic signals respectively stimulating a right inner ear and a left inner ear of the person,

- Figure 3 is a block diagram of the device of Figure 2, illustrating the elements of the device and the functional links between these elements,

- Figure 4 comprises graphs of a physiological signal, a physiological control signal, and the progression of the location in the file of the audio data as a function of time,

- Figure 5 is a diagram of a sound data file,

- Figure 6 is a schematic view of the electronics.

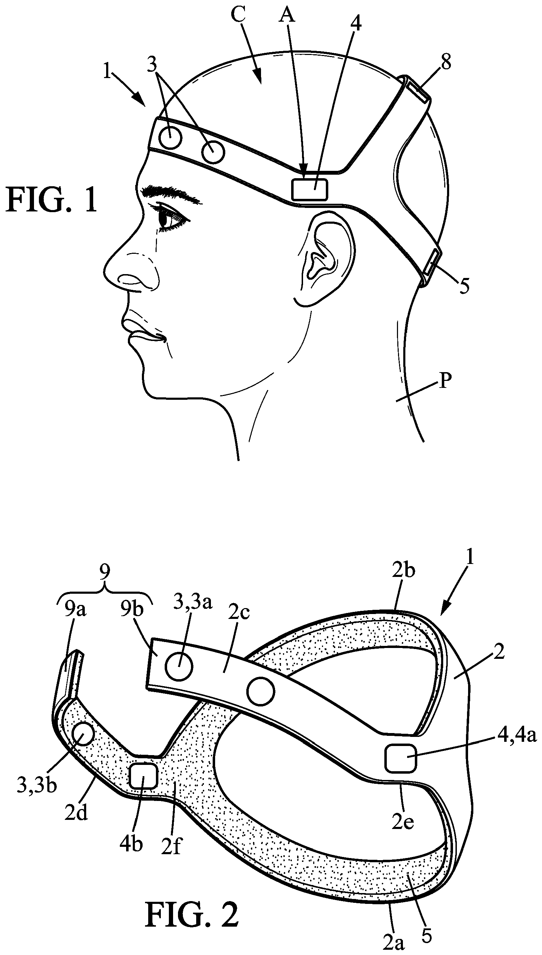

On the figures, identical or similar elements are designated using the same reference sign.
Thereafter, one or more embodiments of the invention will be described.
Referring firstly to Figures 1 and 2, a first object of the invention is a device 1 for stimulating brain waves.
The device 1 is adapted to be worn by a person P, in particular during the person's sleep period.
The device is adapted in particular to be worn on the head of the person P.
To this end, the device 1 comprises a supporting member 2. The supporting member 2 is adapted to surround the head of the person P at least partially so as to be held thereon. In one embodiment of the invention illustrated in Figure 1, the supporting member 2 is particularly adapted to surround at least a portion of a circumference of the head of the person P, in particular surrounding at least half of a circumference of the head of the person P, or even entirely surrounding a diameter of the head of the person P.
In the embodiment illustrated in Figure 1, the supporting member 2 has several arms 2a, 2b, 2c, 2d. The supporting member comprises in particular four arms interconnected at arm connection points 2e, 2f. The arms 2a, 2b, 2c, 2d surround different portions of the head of the person P so as ensure stable retention and a precise positioning of the device 1 on the person P.

For example, a first arm 2a surrounds a back of the head, and a second arm 2b surrounds the top of the head. The first and second arms 2a, 2b are respectively connected at their respective ends at a left lateral arm connection point 2e and a right lateral arm connection point 2f, respectively located near the left and right temples of the person P. Finally, the third and fourth arms 2c, 2d respectively extend from the left lateral 2e and right lateral 2f arm connection points, towards the front of the person P.

The device 1 further comprises a plurality of electrodes 3, at least one acoustic transducer 4, and embedded conditioning and control electronics 5.

The electrodes 3, acoustic transducer 4, and electronics 5 are operatively connected to each other. Thus, the embedded conditioning and control electronics 5 are particularly suitable for controlling and for receiving information from the plurality of electrodes 3, and are also able to command and control the emission of an acoustic signal A by the acoustic transducer 4.

To this end, the electrodes 3, the acoustic transducer 4, and the electronics 5 are mounted on the supporting member 2. In this manner, the electrodes 3, the acoustic transducer 4, and the electronics 5 are close to each other so that communication between these members 3, 4, 5 is particularly fast and high speed. In the example of Figure 1, the electrodes are mounted on the third and fourth arms 2c, 2d, the electronics 5 are mounted on the first arm 2a, and two acoustic transducers 4 are respectively mounted near the left lateral 2e and right lateral 2f arm connection points. Other arrangements of the components of the device 1 are possible.

This allows implementing an operation of stimulating the brain waves of a person P in soft real-time.

Thus, in particular, the electronics 5 are capable, in soft real-time, of receiving a measurement signal S from the plurality of electrodes 3 and controlling the emission by the acoustic transducer of an acoustic signal A synchronized with a predefined temporal pattern T of a brain wave of the person P.

"Synchronized with a predefined temporal pattern of a brain wave" is understood to mean that the acoustic signal emitted by the device is temporally synchronized with a brain wave of the person. More precisely, it means that the acoustic signal emitted by the device is temporally synchronized with an instantaneous phase of a brain wave of the person as detailed below.

"soft real-time" is understood to mean an implementation of the stimulation operation such that the time constraints on this operation, in particular the duration of the operation or the frequency at which it is repeated, are satisfied on the average over a predefined total implementation duration, for example a few hours. It is understood that the implementation of said operation may at certain times exceed said time constraints as long as the average operation of the device 1 and the average implementation of the method satisfies these constraints over the predefined total implementation duration. Time limits may be predefined, beyond which the implementation of the stimulation operation is to be stopped or paused.

To enable such an implementation in soft real-time, a maximum distance between the electrodes 3, the acoustic transducer 4, and the electronics 5 may be less than approximately one meter and preferably less than a few decimeters, enabling them to be connected through a wire embedded in the wearable. In this manner, sufficiently rapid communication between the elements of the device 1 can be guaranteed.

The electrodes 3, the acoustic transducer 4, and the electronics 5 may for example be housed in cavities of the supporting member 2, snapped onto the supporting member 2, or attached to the supporting member 2 for example by gluing, screwing, or other suitable means of attachment. In one embodiment of the invention, the electrodes 3, the acoustic transducer 4, and the electronics 5 may be detachably mounted on the supporting member 2.

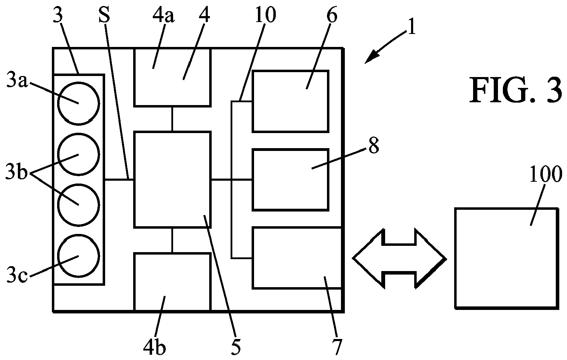

Referring now to Figure 3 as well, in one advantageous embodiment of the invention, the embedded conditioning and control electronics 5 are operatively connected to the electrodes 3 and to the acoustic transducer 4 by means of wire connections 10. In this manner, exposure of the person P to electromagnetic radiation is reduced.

The acoustic transducer 4 is adapted to emit an acoustic signal A stimulating at least one inner ear of the person P.

In a first embodiment illustrated in particular in Figures 1 and 2, the acoustic transducer 4 is an osteophonic device stimulating the inner ear of the person P by bone conduction.

This osteophonic device 4 may for example be adapted for placement near the ear, for example above it as illustrated in Figure 1, in particular on a region of skin covering a cranial bone.

In a second embodiment, the acoustic transducer 4 is a speaker stimulating the inner ear of the person P via an ear canal leading to said inner ear.

This speaker may be placed outside the ear of the person P or in the ear canal.

The acoustic signal A is a modulated signal that at least partially lies within a frequency range audible to a person P, for example the range of 20Hz to 30kHz.

The electrodes 3 are adapted to be in contact with the person P, and in particular with the skin of the person P, in order to capture at least one measurement signal S representative of a physiologic electrical signal E of the person P.

The physiological electrical signal E may in particular be an electroencephalogram (EEG), electro-myogram (EMG), electrooculogram (EOG), electrocardiogram (ECG), plethysmogram, pulse-oxygram and accelerometer, or any other biosignal measurable in a person P.

In particular, the physiological electrical signal E advantageously is an electroencephalogram (EEG) of the person P.

To this end, in one embodiment of the invention, the device 1 comprises at least two electrodes 3 of which at least one is a reference electrode 3a and at least one is an EEG measurement electrode 3b.

The device 1 may further comprise a ground electrode 3c.

In one particular embodiment, the device 1 comprises at least three EEG measurement electrodes 3, so as to capture physiological electrical signals E comprising at least three electroencephalogram measurement channels.

The EEG measurement electrodes 3 are for example arranged on the surface of the scalp of the person P.

In other embodiments, the device 1 may further comprise an EMG measurement electrode, and possibly an EOG measurement electrode.

The measurement electrodes 3 may be reusable electrodes or disposable electrodes. Advantageously, the measurement electrodes 3 are reusable electrodes in order to simplify the everyday use of the device.

The measurement electrodes 3 may be dry electrodes or electrodes coated with contact gel. The electrodes 3 may also be textile or silicone electrodes.

In one embodiment of the invention, the measurement electrodes 3 are active electrodes adapted to preprocess the measurement signal S, for example to perform at least one of the following preprocessing operations:

- frequency filtering, for example frequency filtering of the measurement signal S within a range of temporal frequencies of interest, for example a frequency range within 0.3 Hz to 100 Hz,
- amplification, for example amplification of the measurement signal S by a factor ranging from $10^3$ to $10^6$, and/or
- sampling the measurement signal S by means of an analog-to-digital converter adapted, for example, to sample the measurement signal S at a sampling rate of several hundred Hertz, for example 256 Hz or 512 Hz.

Such preprocessing of the measurement signal S may for example be implemented by an analog module of the measurement electrode 3 or by an analog module located near the measurement electrode 3.

The embedded conditioning and control electronics 5 receive the measurement signals S from the electrodes 3, possibly preprocessed as detailed above.

Alternatively, one may use other kinds of sensors to measure physiological signals of the user. These may include one or more of a pulse-oxymeter and/or inertial sensors. Physiological signals may thus include a movement signal, such as a respiratory movement signal, obtained from an accelerometer, or a signal representative of respiration, such as obtained from a pulse-oxymeter and/or a signal representative of the cardiac rythm. Other considered signals may include body temperature, body sound and/or body vibrations signals.

If the measurement signals S received by the electronics 5 are not preprocessed, the electronics 5 may apply one and/or more preprocessing operations as detailed above.

The embedded conditioning electronics 5 include one or more microchips, for example at least one microprocessor.

As detailed above, the embedded conditioning and control electronics 5 are adapted to implement an operation of stimulating brain waves of the person P.

Said means of the embedded conditioning and control electronics 5 are for example microchips, microprocessors, and/or electronic memories, where appropriate mounted and interconnected on flexible or rigid printed circuit boards and operatively connected to the electrodes 3 and to the transducer 4 via wired connections 10.

The device 1 may further comprise a memory 6 as illustrated in Figure 3. The memory 6 is adapted to be mounted on the supporting member 2, for example as described above for the electrodes 3, the acoustic transducer 4, and the electronics 5. The memory 6 may be permanently mounted on the supporting member 1 or may be a removable module, for example a memory card such as an SD card (acronym for "Secure Digital").

The memory 6 is operatively connected to the electronics 5. The memory 6 may be controlled by the embedded conditioning and control electronics 5 so as to store the measurement signals S.

In one advantageous embodiment of the invention, the memory 6 is capable of storing measurement signals S for a duration of several hours, for example at least eight hours so as to cover an average sleep period of a person P.

The device 1 may further comprise a communication module 7 for communicating with an external server 100. The communication module 7 may be mounted on the supporting member 1 as described above for the electrodes 3, the acoustic transducer 4, and electronics 5. The communication module 7 may be controlled by the embedded conditioning and control electronics 5.

The electronics 5 may in particular be adapted to control the communication module 7 to transfer the measurement signals S stored in memory 6 to an external server 100. The transfer operation may be implemented after a sleep period of the person P.

The communication module 7 may advantageously be a wireless communication module, for example a module implementing a protocol such as Bluetooth or Wi-Fi.

In this manner, when the P person is in a sleep period, he or she is not disturbed by cables, in particular if it is necessary to transmit data during the sleep period.

The device 1 may also comprise a battery 8. The battery 8 may be mounted on the supporting member 1 as described above for the electrodes 3, the acoustic transducer 4, and the electronics 5. The battery 8 may be capable of supplying power to the plurality of electrodes 3, the acoustic transducer 4, and the electronics 5, and where appropriate the memory 6 and the communication module 7. The battery 8 is preferably adapted to supply power for several hours without recharging, more preferably for at least eight hours so as to cover an average sleep period of a person P.

The device 1 can thus operate autonomously during a sleep period of the person P. In this manner in particular, the device 1 is self-contained and adapted to implement one or more operations of stimulating slow brain waves without communicating with an external server 100, in particular without communicating with an external server 100 for several minutes, more preferably several hours, more preferably at least eight hours. This reduces the exposure of the person P to electromagnetic radiation. In particular, the device 1 may also be used to assist the person with falling asleep.

"Self-contained" is thus understood to mean that the device can operate for an extended period of several minutes, preferably several hours, in particular at least eight hours, without needing to be recharged with electrical energy, communicate with external elements such as an external server, or be structurally connected to an external device such as a securing member such as an arm or a bracket.

In this manner the device is suitable for use in the everyday life of a person P without imposing particular constraints.

Furthermore, the supporting member 2 advantageously comprises a device 9 for adjusting to the diameter of the head of the person P. This allows adjusting device 1 to the person P and therefore enables particularly good contact between the electrodes 3 and the skin of the person P.

The adjustment device 9 allows changing a dimension of the supporting member 2 according to a diameter of the head of a person P, to allow fine-tuned adjustment to said diameter.

In one embodiment illustrated in particular in Figure 1, the adjustment device 9 comprises at least two parts 9a, 9b that are movable with respect to one another. The parts 9a, 9b may be rigid or semi-rigid. In the example of Figure 1, the parts 9a and 9b are respectively the ends of the third and fourth arms 2c, 2d of the supporting member 2. The device 1 can be adjusted to and remain in place on the head of the person P.

In a variant of this embodiment, the adjustment device 9 may also include a lock adapted to prevent or allow a relative movement of said two parts 9a, 9b. The lock may be an integral part of one of parts 9a, 9b or may be an element independent of the two parts 9a, 9b.

In another embodiment of the invention, the adjustment device 9 is a soft and flexible portion of the supporting member 2. This portion may be a portion of fabric or elastomer, for example of stretch fabric.

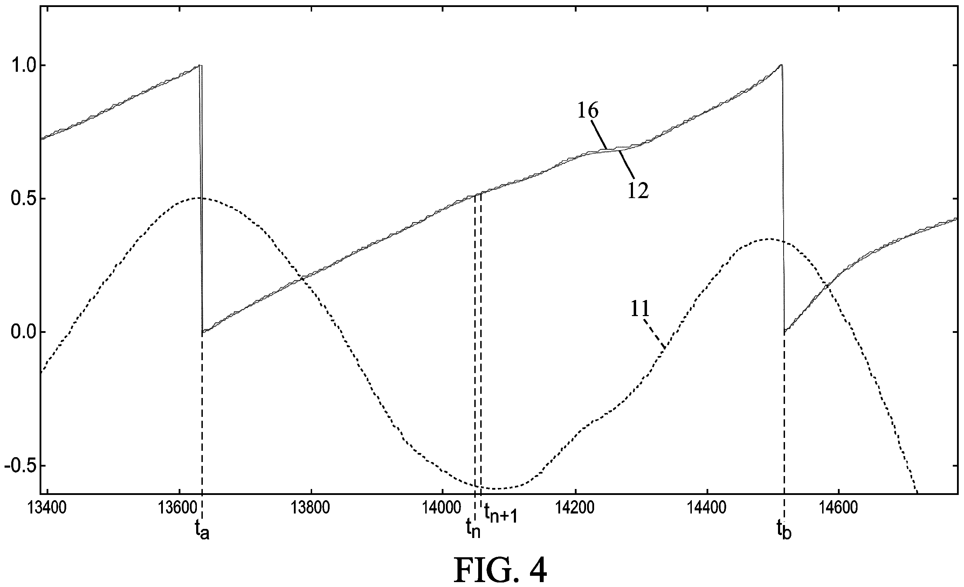

According to the present embodiment, a physiological signal 11 of the user is obtained as a function of time. For example, it is an acceleration signal such as shown on Figure 4. The acceleration signal is representative of the user's breathing cycle. It is for example obtained through filtering the raw acceleration signal in a band of frequencies corresponding to normal user breathing frequency. As can be seen, such a signal is a pseudo-periodic signal representative of the cyclic biological mechanisms taking place into the user's body. According to another example, it is a pulsoxymeter signal representative of the cardiac rhythm.

According to one aspect, one maps the instantaneous phase phi (t) of the physiological signal 11 as a function of time onto a pre-determined interval, for example [0; 1] for each period. This signal is also a physiological signal. The obtained physiological signal is shown on Fig. 4 as the brokenly continuous line 12. To do so, first, the start $t_a$ of a period is detected. For example, the start $t_a$ of a period is detected as equal to the end of the previous period. Provisionally, $t_b$ is defined as $t_a+T$, where T is the pre-defined maximum period value for the physiological signal. For example, T is about 1 to 12 seconds, notably about 4 to 12 seconds for the plethysmogram. The phase signal 12 is continuously monotonous and mapped to [0;1] on a time interval starting from $t_a$. When the phase signal reaches 360°, the actual time $t_b$ is detected. This physiological signal will be used to control the emission of the acoustic waveform. For this reason, it is called a physiological control signal.

One sets an interval of time $\delta t$ which is the time interval for the calculations. At every time $t_n=t_a+n*\delta t$, where n is an incrementally increased integer up to the time where $t_n>t_b$, one defines a time interval $[t_n; t_{n+1}]$ of duration $\delta t$. The order of magnitude for $\delta t$ is for example about 1 to 50 milliseconds (ms). Hence, the ratio of $\delta t$ to $(t_b-t_a)$ is at most 5%, and can be much lower, depending on the kind of physiological signal and the frequency of the calculation, for example at most equal to 1% , at most equal to 0.3%, and notably at most equal to 0.1%.

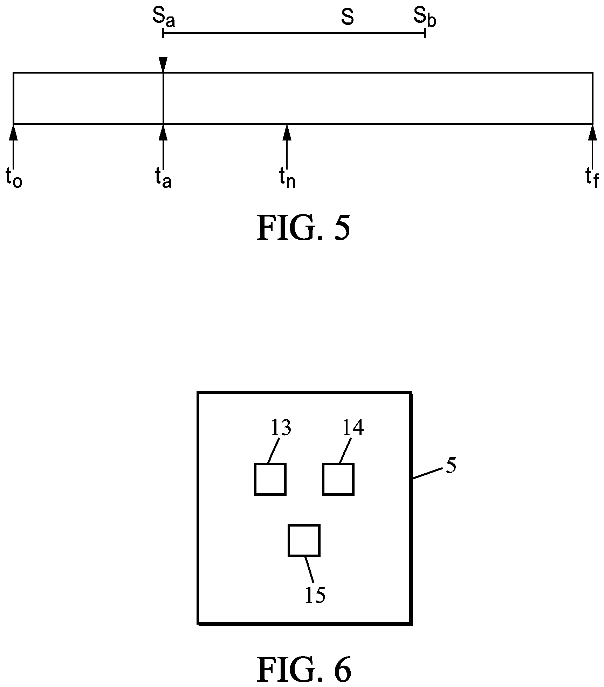

The memory 6 holds sampled sound data in the form of a digital file. The sound data is sufficiently long to be played over a plurality of periods of the physiological signal. For example, the sound data can be played for one or more minutes. Upon reading the sound data file, one progresses from an end $t_0$ to another end $t_f$. In some cases, at $t_f$, one may start again playing the sound data file at $t_0$, proceed to another sound data file, or stop playing sound. The sound data file comprises a given number of samples. Each sample lasts for a time interval $\epsilon t$. $\epsilon t$ is much lower than $\delta t$. A typical sound data file is sampled at about 48 kHz. $\epsilon t$, in such case, is approximately equal to 21 micro-seconds. Thus, typically, the order of magnitude of $\epsilon t$ is 100 to 1000 times lower than that of $\delta t$. For example, the calculation can be performed every 512 read samples. The arrow $t_a$ on Fig. 5 represents the location in the sound data file at time $t_a$. At time $t_a$, one selects a pre-determined maximum number S of samples which may be read during the period $[t_a; t_b]$. The first sample is labeled $S_a$, and the last sample is labeled $S_b$, such that $S_b-S_a=S$.

The arrow $t_n$ on Fig. 5 represents the location in the sound data file at time $t_n$. N is defined as the number of samples which are read during $\delta t$. In the present example, as discussed above, N is set to 512. The function $\psi(t)$ is used to designate the location in the sound file at time t with respect to the maximum number S of samples to be read during the time interval $[t_a; t_b]$. This corresponds to the current rate of reading the sound file at time t. As discussed above $\psi(t_{n+1})= \psi(t_n)+N/S$.

P is defined as the number of sound samples which are going to be played during $\delta t$. Further, R is defined as the speed of playing the sound file (number of played sample per time). As far as possible, R is a pre-defined constant, and R=P/$\delta t$. N is thus larger than P. Indeed, a read sample is either played, or unplayed. The rate $r_n$ of samples to be played during the time interval $\delta t$ is defined as $r_n=P/N$. (N-P) is thus the number of read samples which are not played during $\delta t$.

According to the present embodiment, a rate determination module 13 of a processor determines a rate $r_n$ of samples to be played during that time interval $[t_n; t_{n+1}]$ of duration $\delta t$. In particular, $r_n$ is based on the value phi($t_n$) of the physiological control signal at time $t_n$. In other words, $r_n$, the ratio of played samples to read samples, depends on the phase of the physiological signal. The slower the physiological signal is, the more read samples are played.

It is desired that the location $\psi(t)$ in the data filed be synchronized with the phase of the physiological signal. So, we are targeting $\psi(t_{n+1})= $ phi($t_{n+1}$).

As discussed above, $\psi(t_{n+1})= \psi(t_n)+N/S$.

According to the mathematical definition of the time derivative, phi($t_{n+1}$)= phi($t_n$)+(d.phi/dt).$\delta t$.

It follows from the above three paragraphs that $\psi(t_n)+N/S= \psi(t_{n+1})= $ phi($t_{n+1}$)= phi($t_n$)+(d.phi/dt).$\delta t$.

As discussed above, $\delta t$=P/R.

It follows from the above two equations that $\psi(t_n)+N/S = $ phi($t_n$)+(d.phi/dt).P/R.

Solving for r in that equation, we get:

$$r_n = (\frac{\psi(t_n) - phi(t_n)}{N} + \frac{1}{S}) * \frac{R}{\frac{\partial phi}{\partial t}}$$

As discussed above, since R and S are pre-set, and d.phi/dt at time $t_n$ can be estimated as (phi($t_n$)-phi($t_{n-1}$))/$\delta t_{n-1}$, with $\delta t_{n-1}$ corresponding to the time duration of the previous interval, the rate of played samples for interval $[t_n; t_{n+1}]$ can be determined from this equation.

According to a second embodiment, the duration of the time interval $\delta t$ is set, and N is allowed to vary with time. In such case, expressing N as N=P/$r_n$, one gets:

$$r_n = \frac{P}{S(phi(t_n) - \psi(t_n) + \frac{\partial phi}{\partial t}\delta t)}.$$

Noting that (P/S)=(P/R)*(R/S)= $\delta t$*(R/S),

$$\frac{R}{S} = \left(\frac{\partial \psi}{\partial t}\right)_{(t=0)},$$

and that        the theoric reading rate of the audio file if it was played normally, $r_n$ can be expressed as:

$$r_n = \frac{\left(\frac{\partial \psi}{\partial t}\right)_{(t=0)}}{\left(\frac{phi(t_n) - \psi(t_n)}{\delta t} + \frac{\partial phi}{\partial t}\right)}.$$

A sampling module 14 of the processor samples $P = r_n * N$ samples of the audio file from the N samples which were preliminary selected for the time interval $[t_n; t_{n+1}]$. This may require altering the samples so as to respect a general sound features of the played sound, as is generally known in the sound industry (for example to maintain a pitch comparable with a pitch of previously generated less-compressed samples). This signal forms a control signal which can be sent to a transducer 4 or other equipment to generate a sound. Hence, a command module 15 of the processor commands the play of the P samples (which form a part of the overall played acoustic waveform) from the sampled sound data. This command depends on the determined rate $r_n$ of samples.

Hence, at $t_{n+1}$, the function $\psi(t_{n+1})$ was increased by N/S (ratio of number of read samples during the previous time interval to the total number of pre-selected samples for the pre-determined period T). On figure 4, the function $\psi(t)$ is also represented as a function of time t as step-wise line 16. As can be seen, this closely follows the phi(t) signal line 12.

If the signal phi(t) reaches 1 before the pre-determined $t_b$ (which should always be the case, since $t_b$ is purposefully set as the highest possible period for the physiological process), a new $t_a$ is defined for the next period, and the value $\psi(t_a)$ for the next period is defined as the previously calculated $\psi(t_{n+1})$. In other words, one starts reading the sound data file where we stopped for the previous period.

According to an aspect, a computer program comprises instructions to execute the steps as described above when executed on a computer.

According to another example, the physiological signal could be obtained from an electro-encephalogram signal.

<div align="center">References :</div>

| | | |
|---|---|---|
| device 1 for stimulating slow brain waves | EEG measurement electrode 3b | battery 8 |
| supporting member 2 | ground electrode 3c | adjustment device 9 |
| arms 2a, 2b, 2c, 2d | acoustic transducer 4 | parts 9a, 9b |
| arm connection points 2e, 2f | conditioning and control electronics 5 | wire connections 10 |
| electrodes 3 | memory 6 | rate determination module 13 |
| reference electrode 3a | communication module 7 | sampling module 14 |
| | | command module 15 |

**Claims**

1.  Method for commanding the production of an acoustic waveform based on a physiological control signal, comprising:

    - providing sampled sound data comprising S sound samples stored on a data carrier,
    - repeatedly, for n successive respective time intervals $[t_n; t_{n+1}[$ between an initial time $t_a$ and a final time $t_b$:

        . providing a physiological control signal phi(t) as a function of time, during the current time interval $[t_n; t_{n+1}[$,
        . a rate determination module (13) of a processor determines a rate $r_n$ of samples to be played during that time interval based on a value phi $(t_n)$ of the physiological control signal at time $t_n$,
        . a command module (15) of the processor commands the play of a part of the acoustic waveform from the sampled sound data as a function of the determined rate $r_n$ of samples.

2.  Method for commanding the production of an acoustic waveform according to claim 1, wherein the physiological control signal phi (t) is representative of the phase of a pseudo-periodic physiological signal.

3.  Method for commanding the production of an acoustic waveform according to claim 2, wherein the pseudo-periodic physiological signal is a respiratory signal or an electro-encephalogram signal of a user.

4.  Method for commanding the production of an acoustic waveform according to any of claims 1 to 3, wherein the physiological control signal phi(t) is a strictly monotonous signal along time.

5. Method for commanding the production of an acoustic waveform according to any of claims 1 to 4, wherein the rate $r_n$ of samples to be played is proportional to a speed (R) of number of played samples per time, between the initial time $t_a$ and the final time $t_b$.

6. Method for commanding the production of an acoustic waveform according to any of claims 1 to 5, wherein the rate $r_n$ of samples to be played is proportional to an inverse of d.phi(t)/dt, where an operator (d.X/dt) designates a time derivative of signal X.

7. Method for commanding the production of an acoustic waveform according to any of claims 1 to 6, wherein the command module (15) of the processor re-samples N sound samples of the sound data into P sound samples, where P is lower than N and r=P/N.

8. Method for commanding the production of an acoustic waveform according to any of claims 1 to 7 wherein, at least one time interval, and notably all time intervals, have a time duration $\delta t= t_{n+1}-t_n$ at most equal to 5%, notably at most equal to 1% , preferably at most equal to 0.3%, notably at most equal to 0.1% of $t_b-t_a$.

9. Method for commanding the production of an acoustic waveform according to any of claims 1 to 8, further comprising determining the physiological control signal phi(t) as a function of time based on a measurement by a sensor.

10. Method for commanding the production of an acoustic waveform according to any of claims 1 to 9, wherein the physiological control signal phi(t) is determined from a pseudo-periodic physiological signal of period $t_b-t_a$.

11. Method for commanding the production of an acoustic waveform according to any of claims 1 to 10, wherein the method is repeated for further periods of time until the sample sound data is played.

12. Method for producing an acoustic waveform comprising:

   - applying a method for commanding the production of an acoustic waveform according to any of claims 1 to 11,
   - for each of said successive time intervals, playing a part of the acoustic waveform from the sampled sound data as a function of the determined rate $r_n$ of samples.

13. Computer program comprising instructions for executing the steps of the methods according to any of claims 1 to 12, when the computer program is run on a processor.

14. System for commanding the production of an acoustic waveform based on a physiological control signal, comprising:

   - a data carrier storing sampled sound data comprising S sound samples,
   - a processor configured to repeatedly, for n successive respective time intervals $[t_n; t_{n+1}[$ between the initial time $t_a$ and the final time $t_b$:

      a physiological control signal phi(t) being provided as a function of time, during the current time interval $[t_n; t_{n+1}[$,

      . determine a rate $r_n$ of samples to be played during that time interval based on a value phi ($t_n$) of the physiological control signal at time $t_n$, using a rate determination module (13) of the processor,
      . command the play of a part of the acoustic waveform from the sampled sound data as a function of the determined rate $r_n$ of samples, using a command module (15) of the processor.

15. System for commanding the production of an acoustic waveform according to claim 14 further comprising a wearable device adapted to be worn on the head of a user, housing the processor and data carrier, and comprising sensors to determine the physiological control signal phi(t) as a function of time.

EP 3 415 089 A1

FIG. 1

FIG. 2

10

FIG. 3

FIG. 4

EP 3 415 089 A1

$S_a$          S          $S_b$

$t_o$          $t_a$          $t_n$          $t_f$

## FIG. 5

13      14

5

15

## FIG. 6

Europäisches
Patentamt

European
Patent Office

Office européen
des brevets

# EUROPEAN SEARCH REPORT

Application Number

EP 17 30 5705

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/021662 A1 (RYTHM [FR]) 9 February 2017 (2017-02-09) * page 23, line 26 - page 34, line 1 * ----- | 1-15 | INV. A61B5/0484 A61B5/00 |
| X | WO 2016/028635 A1 (UNIV NORTHWESTERN [US]) 25 February 2016 (2016-02-25) * page 11, paragraph 49 - page 13, paragraph 54 * * page 16, paragraph 3 - page 17, paragraph 4 * ----- | 1-15 | |

TECHNICAL FIELDS
SEARCHED      (IPC)

A61B

The present search report has been drawn up for all claims

| Place of search | Date of completion of the search | Examiner |
|---|---|---|
| Berlin | 28 November 2017 | Trachterna, Morten |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 30 5705

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2017

| Patent document cited in search report | | Publication date | Patent family member(s) | | Publication date |
|---|---|---|---|---|---|
| WO 2017021662 | A1 | 09-02-2017 | FR<br>WO | 3039773 A1<br>2017021662 A1 | 10-02-2017<br>09-02-2017 |
| WO 2016028635 | A1 | 25-02-2016 | EP<br>US<br>WO | 3182890 A1<br>2017304587 A1<br>2016028635 A1 | 28-06-2017<br>26-10-2017<br>25-02-2016 |

EPO FORM P0459

For more details about this annex : see Official Journal of the European Patent Office, No. 12/82

**EP 3 415 089 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016083598 A